# EUROPÄISCHE PATENTSCHRIFT

(11) **EP 1 904 002 B1**
(45) Veröffentlichungstag und Bekanntmachung des Hinweises auf die Patenterteilung: **26.08.2009**
(21) Anmeldenummer: 06761732.4
(22) Anmeldetag: 29.06.2006
(51) Int. Cl.: A61F 5/02

(54) **RUMPFORTHESE**
TRUNK ORTHOSIS
ORTHESE DE TRONC

(30) Priorität: 05.07.2005 DE 102005031867
(43) Veröffentlichungstag der Anmeldung: 02.04.2008
(73) Patentinhaber: Otto Bock HealthCare GmbH, 37115 Duderstadt (DE)
(72) Erfinder: VOLLBRECHT, Matthias, 37412 Herzberg (DE); MÜHLENBEREND, Andreas, 04229 Leipzig (DE); LEHMANN, Anke, S-60240 Norrköping (SE)
(74) Vertreter: Lins, Edgar
(86) Internationale Anmeldenummer: PCT/DE2006/001128
(87) Internationale Veröffentlichungsnummer: WO 2007/003168

(56) Entgegenhaltungen:
- DE-B- 1 197 192
- DE-U1-202004 015 328
- FR-A- 1 104 562
- GB-A- 909 970
- US-B1- 6 419 652

## Beschreibung

Die Erfindung betrifft eine Rumpforthese mit einer zum Umschließen des Rumpfes eines Patienten ausgebildeten Bandage und mit einer mit der Bandage verbundenen Stützeinrichtung, die durch eine Vielzahl von nebeneinander angeordneten und direkt miteinander verbundenen Fingerstäben gebildet ist, die aus einem flexiblen Kunststoff bestehen.

Es ist bekannt, Rumpforthesen zur Stützung und Entlastung der Lendenwirbelsäule einzusetzen. Bekannt ist ferner, dass dabei ganz unterschiedliche Funktionen der Orthese erforderlich werden können. So kann es geboten sein, den Lordosenbereich der Wirbelsäule vollständig zu entlasten, indem er durch die Stützeinrichtung überbrückt wird. Hierbei wird eine weitgehende Immobilisierung der Wirbelsäule bewirkt.

Ferner ist es bekannt, den Lumbalbereich bzw. lumbosakralen Bereich der Wirbelsäule während einer eingeschränkten Beweglichkeit zu stützen. In einer weitergehenden Rehabilitationsphase kann es ggf. nur noch erforderlich sein, eine gewisse Stützwirkung mittels einer Bandage oder einer geringfügig verstärkten Bandage auszuüben.

Durch DE 202 04 747 U1 ist eine Rumpforthese der eingangs erwähnten Art bekannt, die auf eine vielseitige Einsetzbarkeit für die verschiedenen Anwendungsfälle und auf die Anpassung an unterschiedliche Patienten ausgerichtet ist. Neben einem Aufbau der Bandage aus zwei überlappenden Teilbandagen, die eine Anpassung der Bandagenhöhe an den jeweiligen Patienten ermöglichen soll, sind für die Bandage unterschiedliche Stützeinrichtungen vorgesehen. Außer in vorgesehene Taschen einschiebbaren Stützstäben können an der Bandagenanordnung unterschiedliche Stützeinrichtungen in Form eines Rückenstützrahmens zur Überbrückung des Lordosenbereichs (Entlordosierung) oder eine Rückengliederpelotte zur Stabilisierung des bewegbaren Lordosenbereichs befestigt werden. Ggf. kann diese Wirbelsäulenorthese durch eine schalenförmige Bauchpelotte ergänzt werden. Die unterschiedlichen Stützeinrichtungen können dabei mittels Klettbandverschlüssen an der Bandage befestigt und somit in einfacher Weise ausgewechselt werden.

Durch DE 20 2004 015 328 U1 ist eine Lumbalorthese bekannt, bei der eine Pelotte beispielsweise durch Klettbänder lösbar an einer Bandage befestigbar ist. Die Pelotte besteht aus einer Mehrzahl von Querspangen, die durch lösbare Gelenke miteinander verbunden sind. Die Querspangen sind quer zur Wirbelsäule ausgerichtet, wobei jede Querspange einem Wirbel zugeordnet ist. Über die Länge der Wirbelsäule sind die Querspangen somit untereinander angeordnet. Die Enden der Querspangen können durch senkrecht zu ihrer Ausrichtung verlaufende laterale Versteifungselemente verbunden werden, wobei durch schrittweises Entfernen der Versteifungselemente die Steifheit der Pelotte in Längsrichtung der Wirbelsäule sukzessive verringert werden kann.

Durch die Zuordnung der Querspangen zu jeweils einem Wirbel ist die Lumbalorthese jedoch nur für spezielle Einsatzzwecke verwendbar, sodass für verschiedene Einsatzzwecke unterschiedliche Stützeinrichtungen vorgehalten und benutzt werden müssen.

Der vorliegenden Erfindung liegt daher die Aufgabe zugrunde, eine Rumpforthese der eingangs erwähnten Art so auszubilden, dass sie möglichst flexibel einsetzbar ist.

Diese Aufgabe wird erfindungsgemäß mit einer Rumpforthese der eingangs erwähnten Art dadurch gelöst, dass die Fingerstäbe in Längsrichtung der Wirbelsäule ausgerichtet sind und dass wenigstens in einem mittleren Bereich der Bandage ausgebildete Fingerstäbe Befestigungseinrichtungen für entsprechend fingerartig ausgebildete Versteifungsstäbe aufweisen.

Bei der erfindungsgemäßen Rumpforthese ist somit die Stützeinrichtung durch ein mittels der Fingerstäbe aufgebautes Gliedersystem gebildet, das eine hohe Flexibilität in mehrfacher Weise ermöglicht. Die erfindungsgemäße Rumpforthese ist in einfacher Weise zur Überbrückung des Lordosenbereichs (Delordosierung) umfunktionierbar, wenn wenigstens die in dem mittleren Bereich der Bandage ausgebildeten Fingerstäbe Befestigungseinrichtungen für entsprechend fingerartig ausgebildete Versteifungsstäbe aufweisen. Die Versteifungsstäbe sind aber vorzugsweise aus einem geeigneten steifen Kunststoff gebildet und auf die aus Kunststoff bestehenden Fingerstäbe aufschnappbar oder aufschraubbar.

Für eine Therapie nach einem Lumbal-Bandscheibenvorfall kann es somit zweckmäßig sein, zunächst eine weitgehende Immobilisierung durch Überbrückung des Lordosenbereichs vorzunehmen. Bessert sich das Krankheitsbild, können die Versteifungsstäbe unproblematisch entfernt werden, sodass die erfindungsgemäße Rumpforthese mit demselben Aufbau der Fingerstäbe dann zur Stützung des im eingeschränkten Umfang mobilen Lordosenbereichs dienen.

Bevorzugt sind die Fingerstäbe so ausgebildet, dass ihre Länge von der Mitte zu den Enden der Bandage hin abnimmt. Dabei sind wenigstens drei zum Ende der Bandage hin letzte Fingerstäbe gleich lang ausgebildet. Dies ermöglicht die Anpassung der Rumpforthese an unterschiedliche Rumpfumfänge dadurch, dass eine geeignete Anzahl der gleich langen Fingerstäbe von den Enden der Stützeinrichtung abgetrennt werden, um die Orthese für geringere Rumpfumfänge anzupassen. Für diese Anwendung ist es vorteilhaft, wenn die Verbindung der wenigstens drei letzten Fingerstäbe leicht trennbar ausgebildet ist.

Die Fingerstäbe sind mit ihren Mittelstücken miteinander verbunden und weisen beidseitig von dem Mittelstück freie Enden auf.

Vorzugsweise wird die an den Mittelstücken vorgesehene Verbindung der Fingerstäbe miteinander durch schmale Stege gebildet, die eine gewisse Beweglichkeit der Fingerstäbe zueinander ermöglichen, andererseits jedoch eine ausreichende Festigkeit der Stützeinrichtung auch in Umfangsrichtung des Rumpfes gewährleisten.

Die Stützeinrichtung wird bezüglich der Länge der Fingerstäbe für eine vorgegebene Körpergröße des Patienten ausgewählt. Durch die flexiblen Fingerstäbe selbst kann eine Lordosenstützung unter Beibehaltung einer gewissen Beweglichkeit der Lordose erzielt werden.

Bei einer weitgehenden Überwindung der Schmerzen kann es dann vorteilhaft sein, die durch die Fingerstäbe gebildete Stützeinrichtung vollständig von der Bandage zu entfernen und eine vorbeugende Reststabilisierung nur noch mit der Bandage selbst vorzunehmen, wobei diese ggf. durch eingebrachte Verstärkungsmittel eine noch etwas erhöhte Stabilisierungswirkung ausüben kann.

Um die durch die Fingerstäbe gebildete Stützeinrichtung unproblematisch von der Bandage entfernen zu können, ist es zweckmäßig, wenn die Fingerstäbe lösbar an der Bandage angebracht sind, vorzugsweise mittels Klettverbindungen.

Da die durch die Fingerstäbe gebildete Stützeinrichtung vorzugsweise außen an der Bandage angebracht ist, kann ein Spanngurt für die Bandage vorzugsweise an den Fingerstäbe selbst geführt sein, wenn diese mit Führungseinrichtungen für den Spanngurt versehen sind. Die Führungseinrichtungen können einstückig mit den Fingerstützen verbunden sein, also bereits beim Spritzvorgang für die Fingerstäbe mit vorgesehen werden.

In einer bevorzugten Ausführungsform weisen die Fingerstäbe über ihre Länge eine Form auf, mit der sie sich beidseitig, ausgehend von einer Mitte, nach außen verjüngen, dann ein verbreitertes freies Ende für ein komfortables Anliegen der Enden am Körper aufweisen. Die Verjüngung dient der Erhöhung der Elastizität und der Anpassbarkeit an die Lordosenkrümmung, wenn die Versteifungsstäbe nicht eingesetzt sind. Bei eingesetzten Versteifungsstäben findet eine Anpassung an die Lordosenkrümmung nicht statt, da der Lordosenbereich in diesem Fall überbrückt wird.

Die Anpassbarkeit der Stützeinrichtung kann - im Bereich außerhalb der mittigen Fingerstäbe - dadurch verbessert werden, dass die Fingerstäbe durch Federelemente miteinander verbunden sind, die eine Variation der Abstände der Fingerstäbe zueinander - in den Seitenbereichen der Stützeinrichtung - ermöglichen. Wenn für die Verbindung der Fingerstäbe jeweils zwei Federelemente vorgesehen sind, kann auch eine gezielte Schrägstellung der Fingerstäbe zueinander erreicht werden. Demgegenüber können die mittleren Fingerstäbe durch die Mittelstücke starr miteinander verbunden sein und ein einheitliches Mittelstück bilden, das keine Variation des Abstandes der Fingerstäbe zueinander ermöglicht. In analoger Weise können die fingerartig ausgebildeten Versteifungsstäbe zu einem einstückigen Versteifungsteil miteinander verbunden sein.

In einer weiterhin bevorzugten Ausführungsform ist die Bandage mehrteilig mit einem die Stützeinrichtung tragenden Mittelstück und mit zwei miteinander zum Schließen der Bandage verbindbaren Endstücken gebildet. Dabei können zum Einsatz zwischen Mittelstück und den Endstücken Zwischenstücke unterschiedlicher Länge vorgesehen sein, die eine Anpassung der Länge der Bandage an den Rumpf des jeweiligen Patienten ermöglichen. Die erforderliche zugfeste Verbindung zwischen den Teilen der Bandage erfolgt vorzugsweise mittels Klettverschlüssen, wobei die miteinander verbindbaren Enden als flaches, beidseitig mit Klettverschlusselementen versehenes Ende einerseits und maulartig mit das flache Ende beidseitig übergreifenden Innenseiten, das mit entsprechenden Klettverschlussgegenelementen versehen ist, ausgebildet sein können.

Die Erfindung soll im Folgenden anhand von in der Zeichnung dargestellten Ausführungsbeispielen näher erläutert werden. Es zeigen:
- Figur 1: eine Draufsicht auf eine flachliegende Rumpforthese mit Verstärkungselementen für eine Überbrückung des Lordosenbereichs
- Figur 2: eine schematische Darstellung der Verwendung der Rumpforthese gemäß Figur 1
- Figur 3: eine Ansicht der flachgelegten Rumpforthese gemäß Figur 1 ohne Verstärkungsstäbe
- Figur 4: eine schematische Darstellung der Verwendung der Rumpforthese gemäß Figur 3
- Figur 5: eine Ansicht der flachliegenden Bandage der Rumpforthese gemäß Figur 1 und 3 nach der Abnahme der durch die Fingerstäbe gebildeten Stützeinrichtung
- Figur 6: eine schematische Darstellung der Anwendung der Bandage gemäß Figur 5
- Figur 7: eine perspektivische Darstellung einer flachliegenden mehrteiligen (nicht zusammengefügten) Bandage einer Rumpforthese nach einem zweiten Ausführungsbeispiel der Erfindung
- Figur 8: eine Darstellung gemäß Figur 7 für eine für eine geeignete Länge zusammengefügte Bandage
- Figur 9: die Darstellung gemäß Figur 8 mit einer aufgebrachten Stützeinrichtung mit einem durch die Stützeinrichtung geführten Spanngurt
- Figur 10: die Darstellung gemäß Figur 9 für die durch ein Versteifungsteil mittig versteifte Stützeinrichtung.

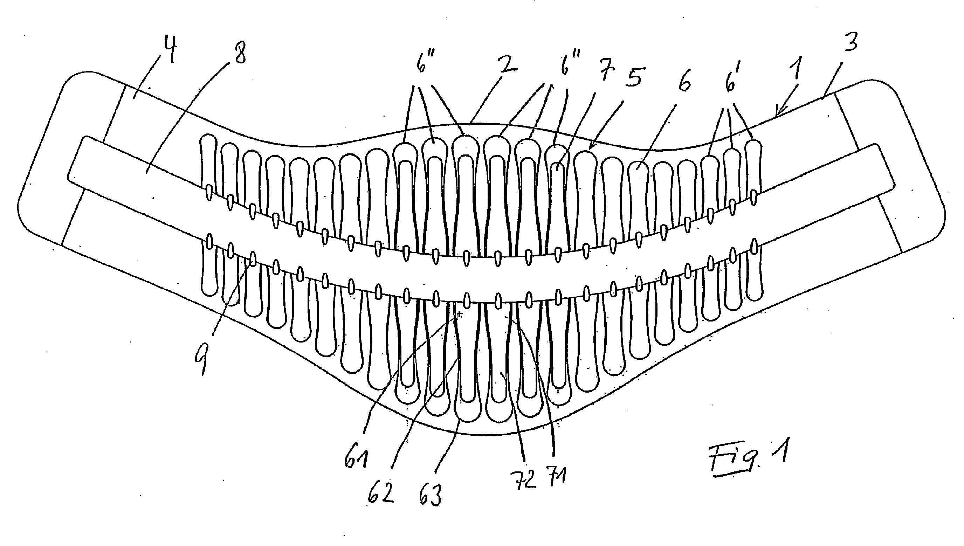

Die in Figur 1 dargestellte Rumpforthese besteht aus einer Bandage 1, die einen mittleren Bereich 2 größerer Höhe aufweist, der in zur Umschlingung des Rumpfes vorgesehene schmalere Endbereiche 3, 4 übergeht, die im ventralen Bereich des Rumpfes überlappend miteinander verbindbar sind, insbesondere durch Klettverbindungen.

Auf der Bandage 1 ist eine Stützeinrichtung 5 lösbar befestigt, die durch parallel zur Wirbelsäule und parallel zueinander und nebeneinander angeordnete Fingerstäbe 6 unterschiedlicher Länge gebildet ist, die über (nicht dargestellte) Stegverbindungen miteinander verbunden sind. In der Mitte der Bandage 1 befindet sich wenigstens ein Fingerstab 6, vorzugsweise zwei Fingerstäbe 6, größter Länge, an den sich zu den Enden 3, 4 hin Fingerstäbe 6 mit abnehmenden Längen anschließen. In dem dargestellten Ausführungsbeispiel sind die drei letzten Fingerstäbe 6' gleich lang ausgebildet und über die Verbindungsstege zueinander leicht voneinander trennbar, sodass die Länge der Stützeinrichtung 5 auf den jeweiligen Rumpfumfang des Patienten angepasst werden kann.

Figur 1 lässt erkennen, dass die sechs mittleren Fingerstäbe 6" der größten Länge durch an den Fingerstäben befestigte Versteifungsstäbe 7 verstärkt sind, wobei die Versteifungsstäbe 7 eine an die Länge des zugehörigen Fingerstabs 6" angepasste (geringere) Länge aufweisen. Ferner ist die Formgebung der Versteifungsstäbe 7 an die Form der Fingerstäbe 6" angepasst.

Die Fingerstäbe 6, insbesondere die Fingerstäbe der größten Länge 6" weisen eine Form auf, die bezüglich ihrer Länge ein breites Mittelstück 61 bildet, an dem die Verbindung zu benachbarten Fingerstäben 6 erfolgt und das in einen sich verjüngenden Abschnitt 62 übergeht, an das sich dann ein wieder verbreitertes freies Endstück 63 anschließt. Demgemäß weisen auch die Versteifungsstäbe ein breites Mittelstück 71 und ein sich daran anschließendes verjüngtes freies Endstück 72 auf.

Die Anbringung der Bandage 1 am Rumpf kann durch einen Spanngurt 8 unterstützt werden, der mittels an die Fingerstäbe 6 angespritzten Führungshaken 9 über die Länge der Bandage 1 im Bereich der Stützeinrichtung 5 geführt wird. Die Führungshaken 9 sind zweckmäßigerweise beim Spritzen der Fingerstäbe 6 einstückig mit diesen hergestellt.

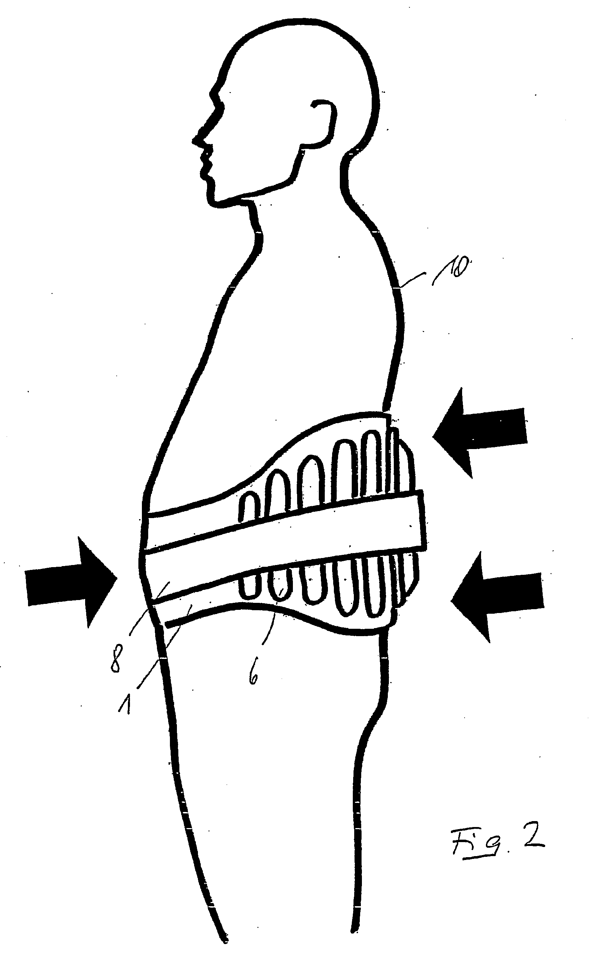

Figur 2 zeigt, dass die in Figur 1 dargestellte Rumpforthese, die mit den im Mittelbereich durch Versteifungsstäbe 7 verstärkten Fingerstäben 6 gebildet ist, zur Überbrückung des Lordosenbereichs eines Patienten 10 verwendet wird, wobei Anlagekräfte gemäß den dargestellten Pfeilen oberhalb und unterhalb des Lordosenbereichs und im Bauchbereich ausgeübt werden.

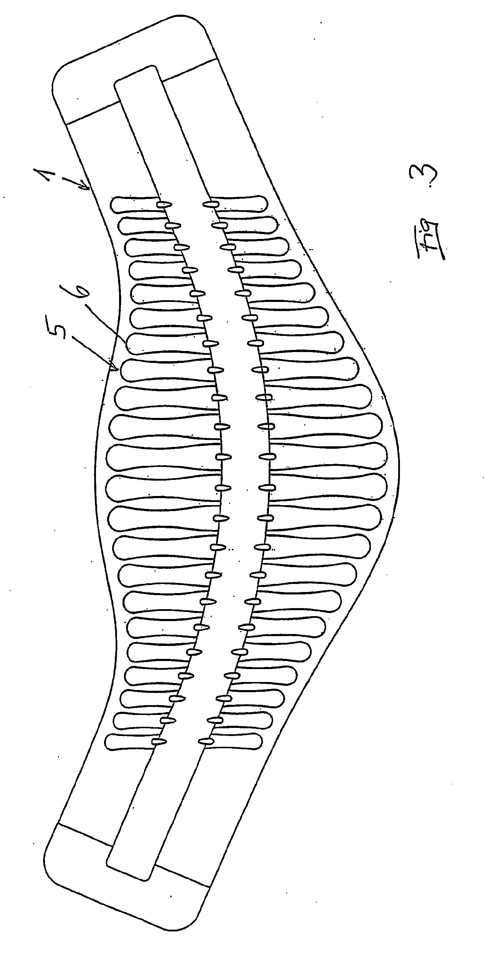

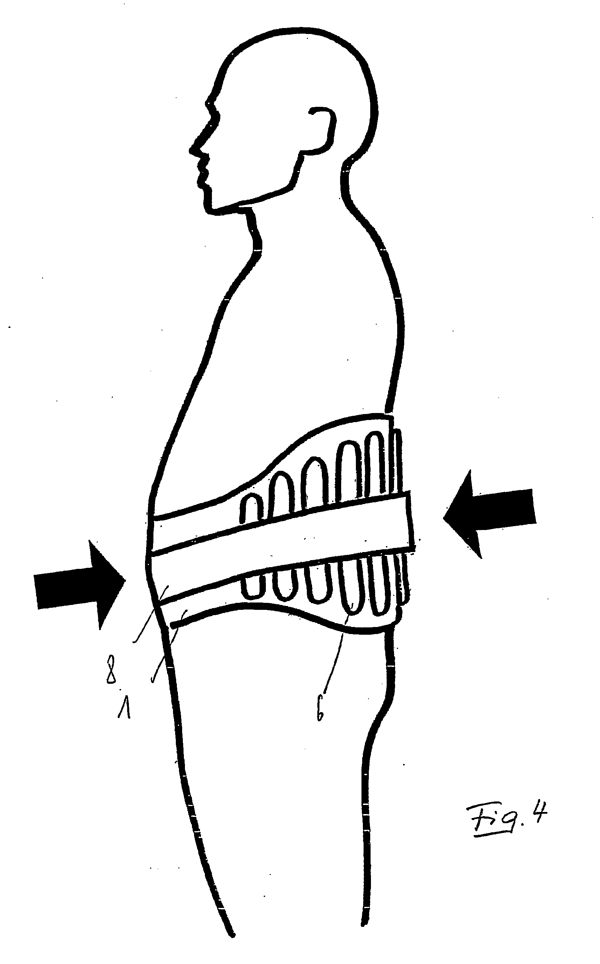

Figur 3 zeigt die Rumpforthese gemäß Figur 1 ohne Versteifungsstäbe 7. Durch das Fehlen der Versteifungsstäbe 7 entfällt die Überbrückungsfunktion für den Lordosenbereich, sodass sich die in ihrer Längsrichtung zu den freien Enden hin flexiblen Fingerstäbe 6 der Krümmung der Wirbelsäule im Lordosenbereich anpassen können, was durch den Spanngurt 8 unterstützt wird. Eine stützende Krafteinleitung erfolgt somit in die Wirbelsäule direkt im Lordosenbereich und in den Bauchbereich, wie dies in Figur 4 angedeutet ist.

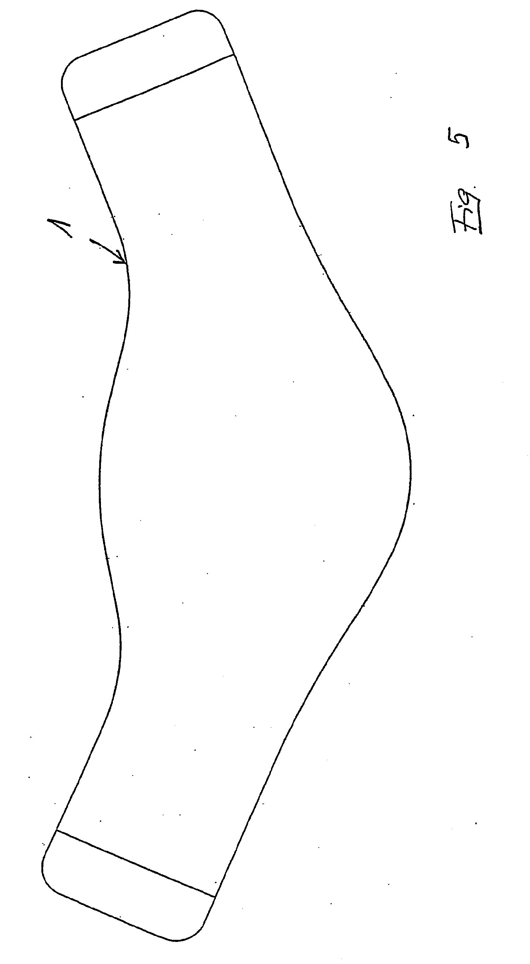

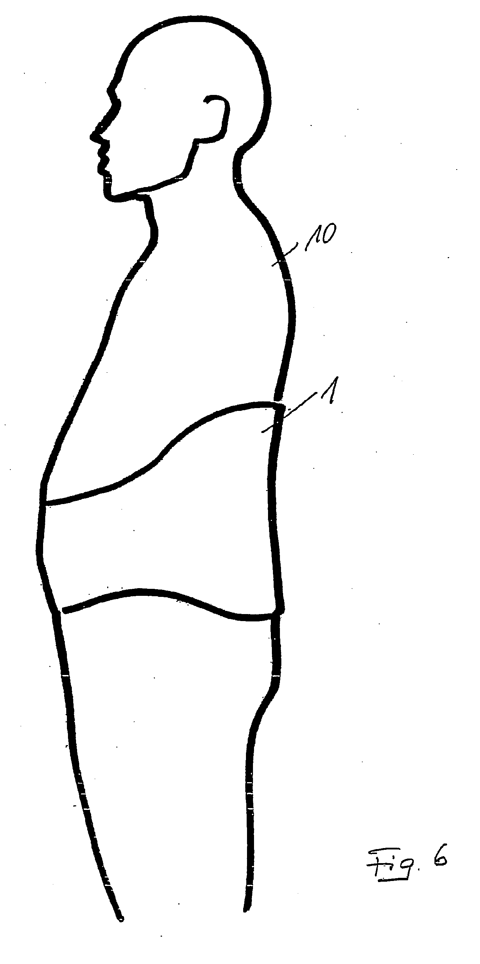

Figur 5 zeigt die Bandage 1 nach dem Entfernen der durch die Fingerstäbe 6 gebildeten Stützeinrichtung 5, beispielsweise durch Lösen einer Klettverbindung. Die Bandage 1 kann dann in ihrer ursprünglichen Form oder ggf. mit eingebrachten oder integrierten Verstärkungselementen für eine generelle Stabilisierung des Patienten 10 im Lumbalbereich verwendet werden, um beispielsweise die Gefahr eines erneuten Bandscheibenvorfalls zu reduzieren (Fig. 6).

Die dargestellte Rumpforthese ist somit in gewissen Maße an den Patienten anpassbar und darüber hinaus in unterschiedlichen Funktionen verwendbar, ohne dass hierfür unterschiedliche Stützeinrichtungen verwendet werden müssten.

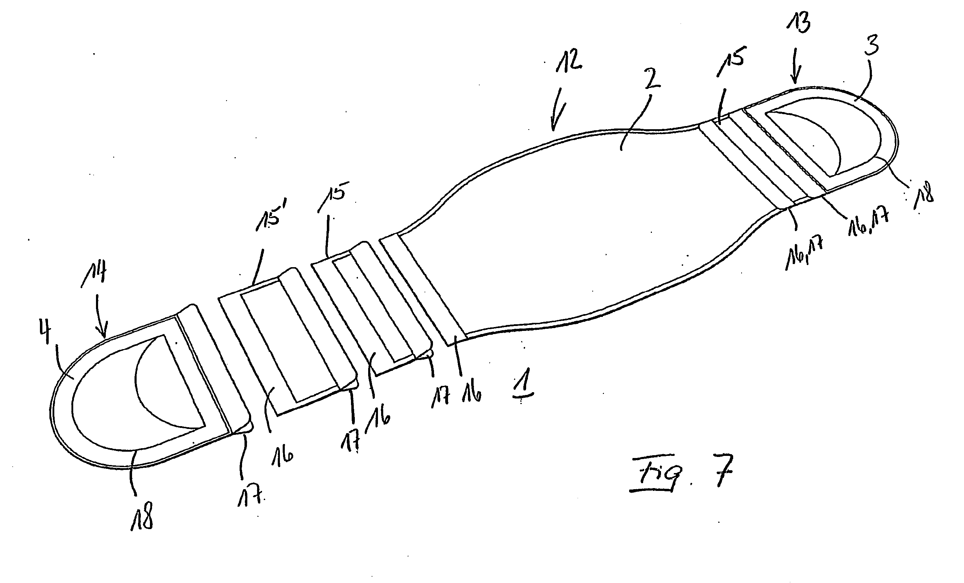

In den Figuren 7 bis 10 ist ein zweites Ausführungsbeispiel einer Rumpforthese dargestellt, dabei ist gemäß Figur 7 die Bandage 1 mehrstückig ausgebildet, wobei ein Mittelstück 12 den breiteren mittleren Bereich 2 und Endstücke 13, 14 die Endbe-reiche 3, 4 bilden. Zwischen dem Mittelstück 12 und den Endstücken 13, 14 ist ein Zwischenstück 15, 15' einsetzbar, wobei Zwischenstücke 15, 15' unterschiedlicher Länge zur Verfügung stehen, um unterschiedliche lange Bandagen 1 zu ermöglichen. In Figur 7 ist zum rechten Endstück 13 ein eingesetztes und mit dem Mittelstück 2 und dem Endstück 13 verbundenes Zwischenstück 15 dargestellt, während auf der linken Seite die zur Verbindung möglichen Zwischenstücke 15, 15' unterschiedlicher Länge einzeln gezeigt sind.

Wenn das zum Bauchumfang des Patienten 10 passende Zwischenstück 15, 15' geeigneter Länge ausgewählt ist, wird es mit dem zugehörigen Endstück 13, 14 und dem Mittelstück 2 zugfest verbunden.

Im dargestellten Ausführungsbeispiel erfolgt die zugfeste Verbindung zwischen dem Zwischenstück 15, 15' und dem Endstück 14 dadurch, dass das Zwischenstück 15, 15' ein zum Endstück 13, 14 zeigendes flaches Ende 16 aufweist, das beidseitig mit Klettverschlusselementen versehen ist. Die Endstücke 13, 14 weisen zu den Zwischenstücken 15, 15' zeigende maulartige Enden 17 auf, die das flache Ende 16 des Zwischenstücks 15, 15' beidseitig übergreifen und mit entsprechenden Klettverschlussgegenelementen versehen sind, sodass beidseitig des flachen Endes 16 eine Klettverschlussverbindung mit den Endstücken 13, 14 hergestellt wird.

In entsprechender Weise ist das Mittelstück 2 mit flachen Enden 16 ausgebildet, die mit zum Mittelstück 12 zeigenden maulartigen Enden 17 des Zwischenstücks 15, 15' zur Herstellung einer zugfesten Klettverbindung 16, 17 zusammenwirken.

Figur 7 lässt noch erkennen, dass die Endstücke 13, 14 an ihrer (in der Darstellung oberen) Außenseite eine aufgesetzte Tasche 18 aufweisen, in die der Patient 10 mit seinen Händen eingreifen kann, um die Bandage 1 um seinen Rumpf herum mit einem gewissen Zug zu befestigen.

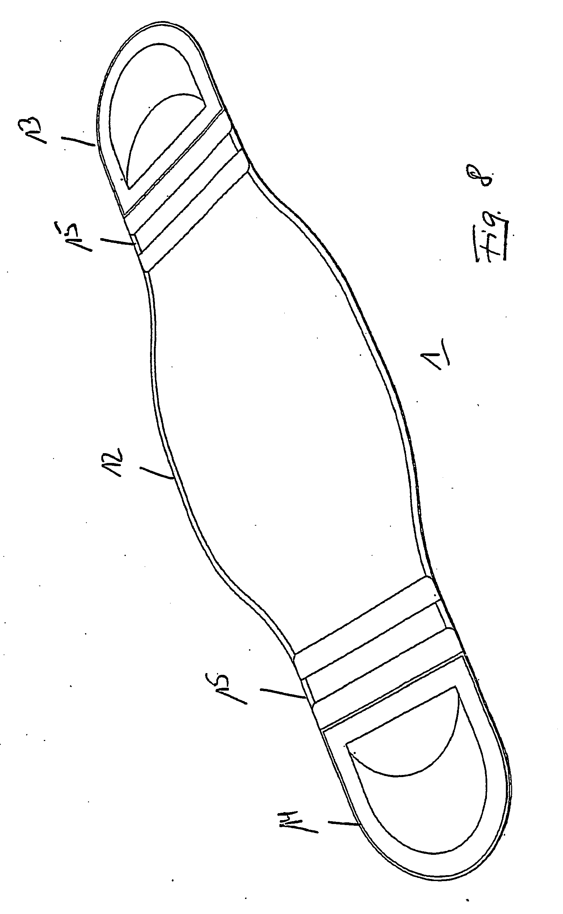

Figur 8 zeigt die nach Auswahl des geeigneten Zwischenstücks 15, 15' zusammengestellte gebrauchsfertige Bandage 1.

Selbstverständlich kann die Bandage 1 bei geringen Körperumfängen auch ohne ein Zwischenstück 15, 15' zusammengestellt werden, indem die Endstücke 13, 14 unmittelbar an dem Mittelstück 12 befestigt werden.

Die in Figur 8 dargestellte Bandage 1 erfüllt die anhand der Figur 6 dargestellte Funktion.

Zur Bereitstellung der Funktion gemäß Figur 4 wird auf die Bandage 1 eine Stützeinrichtung 5' aufgebracht, die aus nebeneinander angeordneten Fingerstäben 26 bestehen, die durch einstückig mit den Fingerstäben ausgebildete Federelemente 27 in ihren Mittenbereichen miteinander verbunden sind. Die Federelemente 27 sind U-förmig ausgebildet, wobei die freien Enden des U als Federschenkel mit dem jeweils benachbarten Fingerstab 26 verbunden ist, sodass eine Variation des Abstandes der benachbarten Fingerstäbe 26 durch ein mehr oder weniger großes Aufspreizen der Schenkel des U-förmigen Federelements 27 ermöglicht wird. Nicht durch Federelemente 27, sondern starr in ihren Mittenbereichen miteinander verbunden sind vier mittlere Fingerstäbe 26', die somit eine in ihrer Längsausrichtung starre Einheit bilden.

Zum Anpressen der Stützeinrichtung 5' an den Lordosenbereich des Patienten 10 dient ein Spanngurt 18, der aus Endelementen 19 an seinen beiden Enden und die beiden Endelemente 19 verbindenden Kunststoff-Drähten 20 besteht, die in einem Laschenstück 21 enden. Die Endelemente 19 sind über Klettverschlüsse mit den Endstücken 13, 14 der Bandage 1 verbindbar. Eine Spannfunktion wird dadurch erreicht, dass die streifenförmigen Endelemente 19 durch eine schlitzförmige Öffnung des zugehörigen Laschenendstücks 21 geführt und um 180 ° umgelenkt sind. In der gespannten Stellung ist das umgelenkte Ende des Endelements 19 ebenfalls mit Hilfe eines Klettverschlusses festlegbar, sodass mit dem Endelement 19 der Spanngurt 18 in einer gewünschten Spannung gehalten wird.

Die parallel verlaufenden Kunststoffdrähte 20 durchlaufen Durchgangsöffnungen in vorstehenden Rippen der Fingerstäbe 26, wobei die mittleren Fingerstäbe 26' ein gemeinsames Mittelstück ausbilden, durch das die Kunststoffdrähte 20 hindurchlaufen. Die beiden parallel zueinander verlaufenden Kunststoffdrähte 20 können im Übrigen durch einen einzigen Kunststoffdraht 20 gebildet sein, der durch die Durchgangsöffnungen der Fingerstäbe 26, 26' gefädelt und in den Laschenendstücken 21 um 180 ° umgelenkt wird, wobei die beiden Enden des Kunststoff-Drahts 20 dann, vorzugsweise mit einem (nicht dargestellten) Verbindungsstück miteinander verbunden werden.

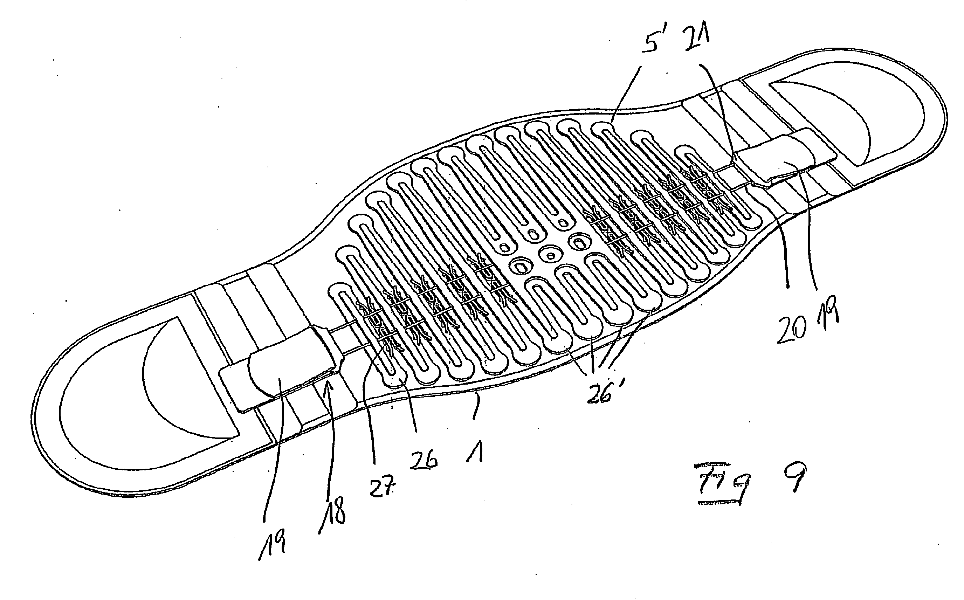

Die in Figur 9 dargestellte Rumpforthese erlaubt eine wirksame Verstärkung des Lordosenbereichs mit einer guten Anpassungsfähigkeit der Stützeinrichtung 5' an die Form des Lordosenbereichs. Hierzu sind insbesondere auch die Fingerstäbe 26, 26' mit einem längeren oberen freien Ende und einem kürzeren unteren freien Ende ausgebildet. Im Übrigen entspricht die Formgebung der Fingerstäbe 26, 26' prinzipiell der anhand der Figur 1 erläuterten Formgebung, wobei die verbreiterten Endstücke 63 dazu dienen, einen möglichst großflächige Anlage am Rumpf des Patienten 10 zu ermöglichen.

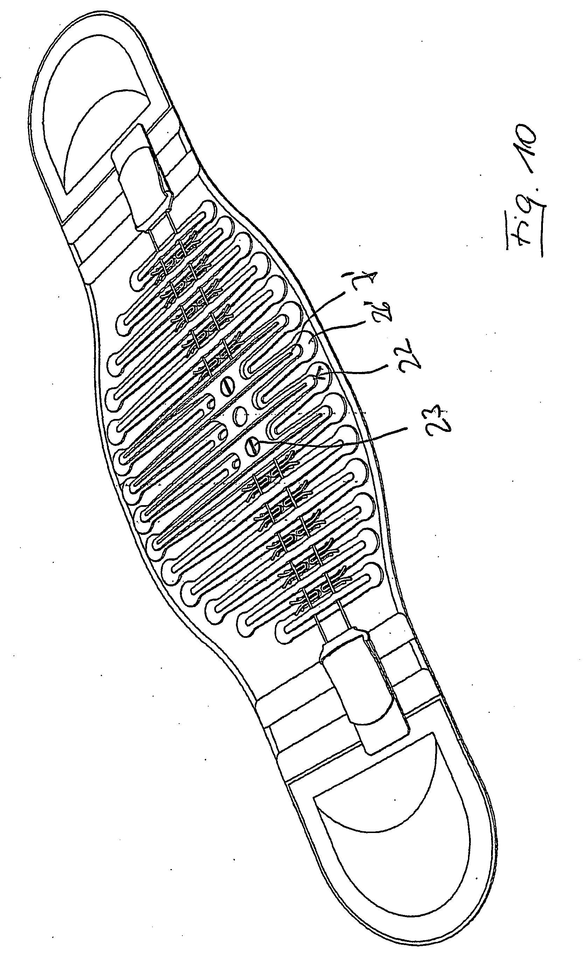

Figur 10 zeigt die Ausführungsform der Erfindung mit einem Versteifungsteil 22, das mittels Verbindungsschrauben 23 auf das mit den mittleren Fingerstäben 26' gebildete Mittelstück der Stützeinrichtung 5' fest aufgebracht ist. Das Versteifungsteil 22 weist an die Form der mittleren Fingerstäbe 26' angepasste Versteifungsstäbe 7' auf, von denen im dargestellten Ausführungsbeispiel vier Versteifungsstäbe 7' vorgesehen sind.

Mit dem Versteifungsteil 22 wird eine elastische Anpassung der Stützeinrichtung 5' an die Lordosenwölbung aufgrund der Elastizität der Fingerstäbe 26 in ihrer Längsrichtung zu den freien Enden hin unterbunden, sodass mit der so gebildeten Rumpforthese gemäß Figur 10 die Stützfunktion gemäß Figur 2 ausgeführt wird, bei der die Lordosenwölbung durch die Rumpforthese überbrückt wird.

Die in den Figuren 7 bis 10 dargestellte Ausführungsform der Erfindung ermöglicht somit die anhand der Figuren 2, 4 und 6 dargestellten Einsatzzwecke, wobei durch den mehrteiligen Aufbau der Bandage 1 eine bequeme Längenanpassung für die Rumpforthese an den Rumpf des Patienten möglich ist und durch den nachträglich befestigbaren Spanngurt ein Andrücken der Stützeinrichtung 5' an den Lordosenbereich unabhängig von der Länge der Bandage 1 ermöglicht wird. Darüber hinaus erlauben die Federelemente 27 eine gute Anpassung der Stützeinrichtung 5' an den Rumpf des Patienten 10 und im Bedarfsfall auch eine gewisse Schrägstellung der Fingerstäbe 26 außerhalb des durch die Fingerstäbe 26' gebildeten mittleren Bereichs.

## Patentansprüche

1. Rumpforthese mit einer zum Umschließen des Rumpfes eines Patienten (10) ausgebildeten Bandage (1) und mit einer mit der Bandage (1) verbundenen Stützeinrichtung (5, 5'), die durch eine Vielzahl von nebeneinander angeordneten und direkt miteinander verbundenen Fingerstäben (6, 26) gebildet ist, die aus einem flexiblen Kunststoff bestehen, **dadurch gekennzeichnet, dass** die Fingerstäbe (6, 26) in Längsrichtung der Wirbelsäule ausgerichtet sind und dass wenigstens in einem mittleren Bereich (2) der Bandage (1) ausgebildete Fingerstäbe (6", 26') Befestigungseinrichtungen für entsprechend fingerartig ausgebildete Versteifungsstäbe (7, 7') aufweisen.

2. Rumpforthese nach Anspruch 1, **dadurch gekennzeichnet, dass** die Länge der Fingerstäbe (6, 26) von der Mitte (2) zu den Enden (3, 4) der Bandage (1) abnimmt.

3. Rumpforthese nach Anspruch 1 oder 2, **dadurch gekennzeichnet, dass** wenigstens drei zum Ende (3, 4) der Bandage (1) hin letzte Fingerstäbe (6') gleich lang ausgebildet sind.

4. Rumpforthese nach Anspruch 3, **dadurch gekennzeichnet, dass** die Verbindung der wenigstens drei letzten Fingerstäbe (6') leicht trennbar ausgebildet ist.

5. Rumpforthese nach einem der Ansprüche 1 bis 4, **dadurch gekennzeichnet, dass** die Verbindung der Fingerstäbe (6, 26) miteinander durch schmale Stege gebildet ist.

6. Rumpforthese nach einem der Ansprüche 1 bis 5, **dadurch gekennzeichnet, dass** die Versteifungsstäbe (7, 7') aus Metall gebildet sind.

7. Rumpforthese nach einem der Ansprüche 1 bis 6, **dadurch gekennzeichnet, dass** die Anordnung der Fingerstäbe (6, 26) lösbar an der Bandage (1) befestigt ist.

8. Rumpforthese nach Anspruch 7, **dadurch gekennzeichnet, dass** die Anordnung der Fingerstäbe (6, 26) mittels Klettverbindungen an der Bandage (1) befestigt ist.

9. Rumpforthese nach einem der Ansprüche 1 bis 8, **dadurch gekennzeichnet, dass** die Fingerstäbe (6, 26) mit Führungseinrichtungen (9) für einen Spanngurt (8, 18) versehen sind.

10. Rumpforthese nach Anspruch 9, **dadurch gekennzeichnet, dass** die Führungseinrichtungen (9) einstückig mit den Fingerstäben (6, 26) verbunden sind.

11. Rumpforthese nach Anspruch 9 oder 10, **dadurch gekennzeichnet, dass** der Spanngurt (18) mittels wenigstens zweier parallel verlaufender Drähte (20) durch die Führungseinrichtungen (9) geführt ist.

12. Rumpforthese nach Anspruch 11, **dadurch gekennzeichnet, dass** der Spanngurt (18) Endelemente (19) aufweist, die mit der Bandage (1) lösbar verbindbar sind.

13. Rumpforthese nach Anspruch 12, **dadurch gekennzeichnet, dass** die Endelemente (19) mittels Klettverschlüssen an der Bandage (1) festlegbar sind.

14. Rumpforthese nach einem der Ansprüche 5 bis 13, **dadurch gekennzeichnet, dass** die die Fingerstäbe (26) verbindenden schmale Stege als Federelemente (27) ausgebildet sind, die eine Variation des Abstands zwischen den Fingerstäben (26) ermöglichen.

15. Rumpforthese nach Anspruch 14, **dadurch gekennzeichnet, dass** die Federelemente (27) einstückig mit den Fingerstäben (26) ausgebildet sind.

16. Rumpforthese nach einem der Ansprüche 1 bis 15, **dadurch gekennzeichnet, dass** die fingerartig ausgebildeten Versteifungsstäbe (7, 7') zu einem ein-stückigen Versteifungsteil (20) miteinander verbunden sind.

17. Rumpforthese nach einem der Ansprüche 1 bis 16, **dadurch gekennzeichnet, dass** die Bandage (1) mehrteilig mit einem die Stützeinrichtung (5') tragenden Mittelstück (12) und mit zwei miteinander zum Schließen der Bandage verbindbaren Endstücken (13, 14) gebildet ist.

18. Rumpforthese nach Anspruch 17, **dadurch gekennzeichnet, dass** zwischen dem Mittelstück (12) und einem Endstück (13, 14) jeweils ein Zwischenstück (15, 15') einsetzbar und mit dem Mittelstück (12) und Endstück (13, 14) zugfest verbindbar ist.

19. Rumpforthese nach Anspruch 18, **dadurch gekennzeichnet, dass** unterschiedlich lange Zwischenstücke (15, 15') zum Einsatz zwischen Mittelstück (12) und Endstück (13, 14) vorgesehen sind.

20. Rumpforthese nach einem der Ansprüche 17 bis 19, **dadurch gekennzeichnet, dass** die Verbindung zwischen Teilen der Bandage (1) mittels Klettverschlüssen erfolgt.

21. Rumpforthese nach Anspruch 20, **dadurch gekennzeichnet, dass** zur Herstellung einer zugfesten Verbindung ein Teil der Bandage (1) ein flaches Ende (16) mit beidseitig angebrachten Klettverschlusselementen und der zu verbindende andere Teil ein das flache Ende (16) beidseitig übergreifendes maulartiges Ende (17) mit Klettverschlussgegenelementen auf den an dem flachen Ende (16) anliegenden Innenseiten aufweist.

## Claims

1. A trunk orthosis with a bandage (1) designed to surround the trunk of a patient (10), and with a support device (5, 5') connected to the bandage (1), the support device (5, 5') is formed by a plurality of finger-shaped rods (6, 26) which are arranged next to one another and connected directly to one another and are made of a flexible plastic **characterized in that,** the finger-shaped rods (6, 26) are oriented in the longitudinal direction of the spinal column and that at least finger-shaped rods (6', 26') positioned in the center (2) of the bandage (1) have securing devices for correspondingly finger-shaped stiffening rods (7, 7').

2. The trunk orthosis as claimed in claim 1, **characterized in that** the length of the finger-shaped rods (6, 26) decreases from the center (2) to the ends (3, 4) of the bandage (1).

3. The trunk orthosis as claimed in claim 1 or 2, **characterized in that** at least three final finger-shaped rods (6') at the end (3, 4) of the bandage (1) are of equal length.

4. The trunk orthosis as claimed in claim 3, **characterized in that** the connection of the at least three final finger-shaped rods (6') is designed to be easily separated.

5. The trunk orthosis as claimed in one of claims 1 through 4, **characterized in that** the connection of the finger-shaped rods (6, 26) to one another is formed by narrow webs.

6. The trunk orthosis as claimed in one of the claims 1 to 5, **characterized in that** the stiffening rods (7, 7') are made of metal.

7. The trunk orthosis as claimed in one of claims 1 through 6, **characterized in that** the arrangement of finger-shaped rods (6, 26) is secured releasably on the bandage (1).

8. The trunk orthosis as claimed in claim 7, **characterized in that** the arrangement of finger-shaped rods (6, 26) is secured on the bandage (1) by means of Velcro® fasteners.

9. The trunk orthosis as claimed in one of claims 1 through 8, **characterized in that** the finger-shaped rods (6, 26) are provided with guide devices (9) for a tightening strap (8, 18).

10. The trunk orthosis as claimed in claim 9, **characterized in that** the guide devices (9) are integrally connected to the finger-shaped rods (6, 26).

11. The trunk orthosis as claimed in claim 9 or 10, **characterized in that** the tightening strap (18) is guided through the guide devices (9) by means of at least two parallel wires (20).

12. The trunk orthosis as claimed in claim 11, **characterized in that** the tightening strap (18) has end elements (19) that can be connected releasably to the bandage (1).

13. The trunk orthosis as claimed in claim 12, **characterized in that** the end elements (19) can be secured on the bandage (1) by means of Velcro® fasteners.

14. The trunk orthosis as claimed in one of claims 5 through 13, **characterized in that** the narrow webs connecting the finger-shaped rods (26) are designed as spring elements (27), which permit a variation in the distance between the finger-shaped rods (26).

15. The trunk orthosis as claimed in claim 14, **characterized in that** the spring elements (27) are designed in one piece with the finger-shaped rods (26).

16. The trunk orthosis as claimed in one of claims 1 through 15, **characterized in that** the finger-shaped stiffening rods (7, 7') are interconnected to form a one-piece stiffening part (20).

17. The trunk orthosis as claimed in one of claims 1 through 16, **characterized in that** the bandage (1) has a multi-part design, with a central piece (12) carrying the support device (5'), and with two end pieces (13, 14) that can be secured to each other to close the bandage.

18. The trunk orthosis as claimed in claim 17, **characterized in that** an intermediate piece (15, 15') can be inserted between the central piece (12) and a respective end piece (13, 14) and can be secured to the central piece (12) and end piece (13, 14).

19. The trunk orthosis as claimed in claim 18, **characterized in that** intermediate pieces (15, 15') of different lengths are provided for insertion between central piece (12) and end piece (13, 14).

20. The trunk orthosis as claimed in one of claims 17 through 19, **characterized in that** the connection between parts of the bandage (1) is obtained by means of Velcro® fasteners.

21. The trunk orthosis as claimed in claim 20, **characterized in that,** in order to produce a strong connection, one part of the bandage (1) has a flat end (16) with Velcro® fastener elements applied on both faces, and the other part to be connected thereto has a mouth-like end (17) which engages over both faces of the flat end (16) and has matching Velcro® fastener elements on the inner faces that bear on the flat end (16).

## Revendications

1. Orthèse de tronc comprenant un bandage (1) formé pour entourer le tronc d'un patient (10) et comprenant, relié au bandage (1), un dispositif de soutien (5, 5') formé par plusieurs barres-doigts (6, 26) agencées les unes à côté des autres et directement reliées les unes aux autres qui sont en une matière synthétique flexible, **caractérisée en ce que** les barres-doigts (6, 26) sont orientées dans la direction longitudinale de la colonne vertébrale et **en ce qu'**au moins des barres-doigts (6", 26') formées dans une région médiane (2) du bandage (1) présentent des dispositifs de fixation pour des barres de rigidification (7, 7') formées conformément comme des doigts.

2. Orthèse de tronc selon la revendication 1, **caractérisée en ce que** la longueur des barres-doigts (6, 26) diminue depuis le milieu (2) jusqu'aux extrémités (3, 4) du bandage (1).

3. Orthèse de tronc selon la revendication 1 ou 2, **caractérisée en ce que qu**'au moins trois dernières barres-doigts (6') vers l'extrémité (3, 4) du bandage (1) sont formées de la même longueur.

4. Orthèse de tronc selon la revendication 3, **caractérisée en ce que** la liaison des au moins trois dernières barres-doigts (6') est formée facilement séparable.

5. Orthèse de tronc selon l'une quelconque des revendications 1 à 4, **caractérisée en ce que** la liaison des barres-doigts (6, 26) entre elles est formée par des traverses étroites.

6. Orthèse de tronc selon l'une quelconque des revendications 1 à 5, **caractérisée en ce que** les barres de rigidification (7, 7') sont en métal.

7. Orthèse de tronc selon l'une quelconque des revendications 1 à 6, **caractérisée en ce que** l'agencement des barres-doigts (6, 26) est fixé au bandage (1) de façon amovible.

8. Orthèse de tronc selon la revendication 7, **caractérisée en ce que** l'agencement des barres-doigts (6, 26) est fixé au bandage (1) au moyen de liaisons Velcro®.

9. Orthèse de tronc selon l'une quelconque des revendications 1 à 8, **caractérisée en ce que** les barres-doigts (6, 26) sont munies de dispositifs de guidage (9) pour une ceinture de serrage (8, 18).

10. Orthèse de tronc selon la revendication 9, **caractérisée en ce que** les dispositifs de guidage (9) sont reliés d'une seule pièce aux barres-doigts (6, 26).

11. Orthèse de tronc selon la revendication 9 ou 10, **caractérisée en ce que** la ceinture de serrage (18) est guidée à travers les dispositifs de guidage (9) au moyen d'au moins deux câbles (20) s'étendant parallèlement.

12. Orthèse de tronc selon la revendication 11, **caractérisée en ce que** la ceinture de serrage (18) présente des éléments d'extrémité (19) qui sont reliables de façon amovible avec le bandage (1).

13. Orthèse de tronc selon la revendication 12, **caractérisée en ce que** les éléments d'extrémité (19) sont fixables au bandage (1) au moyen de fermetures Velcro®.

14. Orthèse de tronc selon l'une quelconque des revendications 5 à 13, **caractérisée en ce que** les traverses étroites reliant les barres-doigts sont formées en tant qu'éléments formant ressort (27), qui permettent une variation de la distance entre les barres-doigts (26).

15. Orthèse de tronc selon la revendication 14, **caractérisée en ce que** les éléments formant ressort (27) sont formés d'une seule pièce avec les barres-doigts (26).

16. Orthèse de tronc selon l'une quelconque des revendications 1 à 15, **caractérisée en ce que** les barres de rigidification (7, 7') formées comme des doigts sont reliées ensemble pour former une partie de rigidification (22) d'une seule pièce.

17. Orthèse de tronc selon l'une quelconque des revendications 1 à 16, **caractérisée en ce que** le bandage (1) est formé en plusieurs parties avec une pièce médiane (12) portant le dispositif de soutien (5') et avec deux pièces d'extrémité (13, 14) reliables ensemble pour fermer le bandage.

18. Orthèse de tronc selon la revendication 17, **caractérisée en ce qu'**à chaque fois une pièce intermédiaire (15, 15') est insérable entre la pièce médiane (12) et une pièce d'extrémité (13, 14), et reliable à la pièce médiane (12) et la pièce d'extrémité (13, 14), de façon résistante à la traction.

19. Orthèse de tronc selon la revendication 18, **caractérisée en ce que** des pièces intermédiaires (15, 15') de différentes longueurs sont prévues pour l'insertion entre pièce médiane (12) et pièce d'extrémité (13, 14).

20. Orthèse de tronc selon l'une quelconque des revendications 17 à 19, **caractérisée en ce que** la liaison entre des parties du bandage (1) s'effectue au moyen de fermetures Velcro®.

21. Orthèse de tronc selon la revendication 20, **caractérisée en ce que** pour fabriquer une liaison résistante à la traction, une partie du bandage (1) présente une extrémité plane (16) comprenant des éléments de fermeture Velcro® rapportés de chaque côté, et l'autre partie à relier présente une extrémité (17) comme une mâchoire recouvrant de chaque côté l'extrémité plane (16) avec des éléments de fermeture Velcro® sur les faces intérieures appliquées sur l'extrémité plane (16).
